# EUROPEAN PATENT SPECIFICATION

(11) **EP 2 166 982 B1**
(45) Date of publication and mention of the grant of the patent: **09.11.2016**
(21) Application number: 08794508.5
(22) Date of filing: 16.07.2008
(51) Int. Cl.: A61F 2/00, A61B 17/04, A61B 17/42

(54) **SURGICAL DEVICES FOR TREATING PELVIC CONDITIONS**
CHIRURGISCHE VORRICHTUNGEN ZUR BEHANDLUNG VON BECKENERKRANKUNGEN
DISPOSITIFS CHIRURGICAUX POUR TRAITER DES TROUBLES DU PELVIS

(30) Priority: 16.07.2007 US 949930 P
(43) Date of publication of application: 31.03.2010
(60) Divisional application: 16188283.2
(73) Proprietor: AMS Research Corporation, Minnetonka, MN 55343 (US)
(72) Inventor: PETERSON, Steven Neil, Minnetonka, Minnesota 55343 (US)
(74) Representative: Vossius & Partner Patentanwälte Rechtsanwälte mbB
(86) International application number: PCT/US2008/008648
(87) International publication number: WO 2009/011852

(56) References cited:
- WO-A-2007/014241
- WO-A-2007/059306
- US-A1- 2003 212 305
- US-A1- 2005 256 366
- US-A1- 2006 089 524
- US-A1- 2007 010 830

## Description

### FIELD OF THE INVENTION

The invention relates to tools and implantable devices; combination, kits, sand systems thereof for treating pelvic conditions. The pelvic conditions include conditions of the female or male anatomy, and specifically include treatments of female or male urinary and fecal incontinence, treatment of female vaginal prolapse conditions including enterocele, rectocele, cystocele, vault prolapse, treatments of conditions of the pelvic floor, and any of these conditions in combination.

### BACKGROUND

Pelvic health for men and women is a medical area of increasing importance, at least in part due to an aging population. Examples of common pelvic ailments include incontinence (fecal and urinary), pelvic tissue prolapse (e.g., female vaginal prolapse), and conditions of the pelvic floor. Urinary incontinence can further be classified as including different types, such as stress urinary incontinence (SUI), urge urinary incontinence, mixed urinary incontinence, among others. Other pelvic floor disorders include cystocele, rectocele, enterocele, and prolapse such as anal, uterine and vaginal vault prolapse. Still others relate to the pelvic floor and are due to weakness or trauma of pelvic floor muscles such as the levator ("levator ani") or coccygeus muscle (collectively the pelvic floor).

May varieties of implants and surgical tools have been proposed and used to address pelvic ailments. Implants are generally placed in contact with pelvic tissue and in a position to support the pelvic tissue. Implants include a portion that contacts the tissue to be supported (e.g., a "tissue support portion") and also include extension portions (also referred to as "end portions," "arms," or "legs"). Some implants have used "connectors" to connect an end of an extension portion to a surgical insertion tool. See for example US patent application 2007/0068538.

### SUMMARY

In one respect the invention involves a connector associated with end of an extension portion. The connector is associated with the extension portion, meaning, for example, that the connector may be attached directly to the extension portion or may be attached indirectly to the extension portion, such as through another structure, e.g., a suture, sheath, or the like. The connector is designed to engage two different tools, e.g., an insertion tool and a removal tool. One tool engages one end of the connector to allow a surgeon to pass the connector through an incision leading to the pelvic region of a patient and place the connector within the pelvic region. The other tool engages a second end of the connector and allows the connector to be passed through tissue, such as through a tissue path leading to an external incision.

The connector and tools can be useful with implants designed to treat any pelvic condition, such as prolapse, incontinence, conditions of the pelvic floor, etc. The use of an insertion tool as described can be particularly useful for treating conditions that require placement of an implant or an extension portion at a "deep" location in the pelvic region, e.g., at a posterior, or an otherwise difficult-to-reach location of a patient's pelvic region. For example, certain methods of treating female posterior prolapse conditions require making an engagement between a connector and a surgical tool in a deep posterior location of the pelvic region such as through a vaginal incision and at the rectovaginal or the pararectal space. The use of a tool that engages the connector and allows the connector to be passed through the vaginal incision, instead of using a surgeon's fingers and hand, can improve manipulation of the connector in the small and difficult-to-access space and may reduce the trauma to the patient by reducing the required degree of dissection.

The use of an insertion tool can generally improve a surgeon's ability to quickly and effectively connect the connector to a tool that allows further manipulation of the connector (e.g., a removal tool), during a pelvic floor repair. The length of an insertion tool, and the configuration of the distal end of the insertion tool (e.g., its shape or angle) can be designed to easily place a connector at a desired location internal to a patient, for engagement with a second tool. Due to the ease of placement of the connector, the insertion and connection process can be performed more quickly and with reduced or minimal dissection and trauma to the patient.

An engagement between a tool and a connector can be any of a variety of mechanical connections, such as by friction between smooth surfaces, by snapfitting, by a threaded engagement, by use of a mechanical detent and corresponding surface structure (e.g., surface depression), etc. According to certain embodiments of tools and connectors, the nature of the engagements between an insertion tool and the connector and a removal tool and the connector allows the connector to be transferred from an engagement with the end of the insertion tool, to an engagement with the end of the removal tool. For embodiments of tools and connectors that are frictional (e.g., non-threaded, non-grasping) and can be disengaged by pulling the connector away from the tool, the force of the engagement between the connector and one of the tools can be lower than the force of the engagement between the connector and the second tool. This allows the connector to be passed (transferred) from one tool to the other by engaging the connector to the first tool, engaging the connector to the second tool, then removing the engagement with the first tool (this engagement having the lower force of engagement).

The invention relates to combination that includes a pelvic implant, an insertion tool, and a removal tool, in accordance with the appended independent claim 1. The implant includes a tissue support portion, an elongate extension portion having a proximal end attached to the tissue support portion and a distal end, and a connector associated with the distal end, the connector comprising a connector proximal end and a connector distal end. The insertion tool includes an elongate shaft and a distal end configured to engage an end of the connector, and the removal tool includes a distal end configured to engage an end of the connector.

The implant includes a tissue support portion, an elongate extension portion having a proximal end attached to the tissue support portion and a distal end, and a connector associated with the distal end, the connector comprising a connector proximal end and a connector distal end. The insertion tool includes an elongate shaft and a distal end configured to engage an end of the connector. The removal tool includes a distal end configured to engage an end of the connector.

The invention also relates to an insertion tool comprising a handle, a shaft, and an end, the end including an elongate cylinder.

### BRIEF DESCRIPTION OF THE DRAWINGS

Figure 1 is a side view illustration of an insertion tool.
Figure 2 is a side-perspective view illustration of an insertion tool.
Figure 3 is an illustration of an insertion tool and portion of an implant.
Figures 4A and 4B are illustrations of relevant anatomy exemplary steps of using tools and connectors as described.
Figure 5 is a side-perspective view of a connector.
Figures 6A, 6B, and 6C are side, cut-away views of a connector, insertion tool, and removal tool.

All figures are schematic, and not necessarily to scale.

### DETAILED DESCRIPTION

The invention relates to surgical instruments, assemblies, combinations (e.g., of implants and tools), and implantable articles for treating pelvic floor disorders such as prolapse (e.g., vaginal prolapse), incontinence (urinary and fecal incontinence), conditions of the pelvic floor such as the perineal body, conditions of levator muscle (such as a component of levator muscle), conditions of the levator hiatus, and combinations of two or more of these. A surgical implant can be used to treat a pelvic condition, wherein the exemplary method includes placing an implant in a manner to support tissue of the pelvic region in a male or female. Exemplary methods involve the use of an implant that includes a connector, and two surgical tools, each of which engages the connector, and can do so at the same time. One tool engages the connector, places the connector at a desired internal location in the pelvic region, transfers the connector to the second tool, and the second connector then manipulates the connector as desired to place the implant at a therapeutic location

An example of a useful set of tools can include one tool that is referred to as an insertion tool, that engages one end of the connector and a second tool referred to as removal tool that engages another end of the connector. The insertion tool generally inserts or otherwise places the connector at a desired location for engagement with the removal tool. The removal tool accepts the connector from the insertion tool and manipulates as desired, especially by passing the connector through tissue, preferably (but not necessarily) through a tissue path that leads through internal tissue and to and through an external incision.

An implant can include a tissue support portion (or "support portion") that can be used to support pelvic tissue such as the bladder or urethra (which includes any location of the bladder, urethra, bladder neck, mid-urethra, or proximal end of the urethra), vaginal tissue (anterior, posterior, vault, etc.), tissue of the perineum, coccygeus, levator ani, levator hiatus, rectum, etc., as desired. During use, the tissue support portion is typically placed in contact with and optionally attached to tissue to be supported, such as with a suture, biological adhesive, mechanical attachment, or any other mode of attachment. An implant can additionally include one or more extension portion (otherwise known as "end" portions or "arms") attached to the tissue support portion. Examples of pelvic implants are described in the following exemplary documents: United States patent number 7,070,556; United States patent number 7,229,453; United States patent number 6,652,450; United States patent number 6,612,977; United States patent number 6,702,827; United States patent publication numbers 2004/0039453; 2005/0245787; 2006/0195011; 2006/0195010; 2006/0235262; 2006/0287571; 2006/0195007; 2006/0260618; 2006/0122457; 2005/0250977; International patent application number PCT/US2006/028928, having an International Filing Date of July 25, 2006, now published as International patent publication WO 2007/016083; Intemational patent application number PCT/US2007/016760, having an International Filing Date of July 25, 2007, now published as International patent publication WO 2008/013867; International patent application number PCT/US2007/014120, having an International Filing Date of June 15, 2007, now published as International patent publication WO 2007/149348 ; and International patent publication WO 2007/097994. Further examples of pelvic implants are described in WO 2007/059306 A, US 2007/010830 A1**,** WO 2007/014241 A and US 2003/212305 A1.

The two part form of independent claim 1 is based on WO 2007/059304

An implant may include portions or sections that are synthetic or of biological material (e.g., porcine, cadaveric, etc.). Extension portions may be, e.g., a synthetic mesh such as a polypropylene mesh. The tissue support portion may be synthetic (e.g., a polypropylene mesh) or biologic. Examples of implant products that may be similar to those useful according to the present description, optionally modified to include a connector as described herein, used with tools as described, include those sold commercially by American Medical Systems, Inc., of Minnetonka MN, under the trade names Apogee® and Perigee® for use in treating pelvic prolapse (including vaginal vault prolapse, cystocele, enterocele, etc.), and Sparc®, Bioarc®, Monarc®, and AdVance™, for treating urinary incontinence.

Exemplary implants can include a tissue support portion for placing in contact with tissue to be supported and one or more "extension" portion, the tissue support portion being useful to support a specific type of pelvic tissue such as the urethra, bladder (including the bladder neck), vaginal tissue (anterior, posterior, apical, etc.), perineum, rectum, levator ani, coccygeus, tissue of the pelvic floor, or other tissue of the pelvic region. The tissue support portion can be sized and shaped to contact the desired tissue when installed, e.g., as a "sling" or "hammock," to contact and support pelvic tissue. A tissue support portion that is located between two or more extension portions is sometimes referred to herein as a "central support portion" or a "support portion."

Extension portions are elongate pieces of material that extend from the tissue support portion and either are or can be connected to the tissue support portion, and are useful to connect to or through tissue of the pelvic region to thereby provide support for the tissue support portion and the supported tissue. One or multiple (e.g., one, two, or four) extension portions can extend from the tissue support portion as elongate "ends," "arms," or "extensions," useful to attach to tissue in the pelvic region.

An example of a particular type of pelvic implant is the type that includes supportive portions including or consisting of a central support portion and either two, four, or six elongate extension portions extending from the central support portion. An implant that has exactly two extension portions can be of the type useful for treating, e.g., urinary incontinence, anterior vaginal prolapse, or posterior vaginal prolapse. An implant having four or six extension portions can be useful for treating anterior vaginal prolapse, or combinations of conditions. The term "supportive portions" refers to extension portions and tissue support portions and does not include optional or appurtenant features of an implant or implant system such as a sheath, connector, or the like.

Examples of implants for treating incontinence, e.g., urethral slings, can include a central support portion and two extension portions, and may take the form of an integral mesh strip. An exemplary urethral sling can be an integral mesh strip with supportive portions consisting of or consisting essentially of a central support portion and two extension portions. Examples of urethral slings for treating male urinary incontinence can have a widened central support portion, as discussed, for example, in Assignee's copending United States patent publication numbers 2006/0287571 and 2006/0235262, Other exemplary urethral sling implants are described in Assignee's United States patent number 7,070,556; United States publication numbers 2006/0195010 and 2006/0195007; and International application numbers WO 2007/097994 and WO 2007/014120.

Examples of implants for treating vaginal prolapse can comprise a central support portion and from two to four to six extension portions, and may take the form of an integral piece of mesh or multiple pieces of mesh attached in a modular fashion. See, e.g., Assignee's copending United States patent publication numbers 2006/0260618; 2005/0245787; 2006/0122457; 2005/0250977; and International patent application number PCT/2006/028828, now published as WO 2007/016083.

Examples of implants for treating conditions of the pelvic floor, such as to support tissue of the perineal body, to treat levator avulsion, to treat levator ballooning, to support or repair levator ani muscle, to tighten or reduce the size of levator hiatus, to treat vaginal prolapse, or to treat fecal incontinence, may take the form of an integral piece of mesh or multiple pieces of mesh attached in a modular fashion. See, e.g., International patent application number PCT/US2007/016760, filed July 25, 2007, by Kimberly Anderson, entitled SURGICAL ARTICLES AND METHODS FOR TREATING PELVIC CONDITIONS, now published as WO 2008/013867.

A length of an extension portion can optionally be fixed (i.e., the extension portion does not include any form of length-adjusting mechanism). Alternate implants may include adjustment or tensioning mechanisms that allow a physician to alter the length of an extension portion before, during, or after implantation. See, e.g., International application number PCT/US2007/014120, filed June 15, 2007, by Dockendorf et al., titled SURGICAL IMPLANTS, TOOLS, AND METHODS FOR TREATING PELVIC CONDITIONS, now published as WO 2007/149348.

According to specific embodiments of implants, various additional components and features can be incorporated for added utility or convenience, such as components and features that facilitate surgical implantation. For instance, a tensioning member (e.g., suture) may be attached to an implant along a portion or entire length of an extension portion for use in adding tension or in positioning an implant or a portion (e.g., extension portion) of an implant. A tensioning suture may be attached at one or multiple attachment points along a length of an end portion. Multiple sutures may be used, such as two or more sutures along a length of one extension portion, for added tensioning effect. Alternately or in addition, extension portions of an implant can include reinforcement or multiple layers. See, e.g., Assignee's copending United States Patent applications US 2006 195011 A1 and US 2006 195010 A1. Other embodiments of the invention do not require and can specifically exclude a tensioning member such as a suture, multiple layers for end portions, and edge extension reinforcement for end portions.

Yet another optional component of an implant can be a sheath such as a flexible, plastic, transparent elongate tube (or "envelope" or "sleeve") that can cover a portion or entire length of an extension portion. A sheath can reduce friction between the implant material and tissue of a tissue path, to facilitate introduction of the implant material to tissue. A sheath may also facilitate installation by allowing a surgeon to apply tension or pressure on the sheath, optionally to indirectly pressure or tension the extension portion or tissue support portion.

An implant according to the invention includes at least one connecter associated with an extension portion. The connector is "associated with" the extension portion, meaning that the connector is directly or indirectly connected or attached to the extension portion, preferably at or near a distal end of the extension portion. The connector may be attached directly to the extension portion, e.g., at a distal end, or may be attached indirectly to the extension portion (e.g., at a distal end) such as through another structure, e.g., a suture, sheath, or the like.

The connector engages two different tools, which may be an insertion tool and a removal tool. The ability to engage two different tools allows the connector to be manipulated using both of the tools, separately or together. In particular embodiments of the invention, each tool engages one end of the connector. One example of a useful tool can be an insertion tool that engages an end of a connector and allows a surgeon to use the insertion tool to pass the connector through an incision leading to the pelvic region of a patient and place the connector within the pelvic region. Another example of a useful tool can be a removal tool that engages an end of the connector and allows the removal tool to pass (e.g., pull) the connector through tissue, such as through a tissue path leading to an external incision.

The two different engagements between one tool (e.g., insertion tool) and the connector, and the second tool (e.g., removal tool) and the connector, should allow the connector to be transferred from an engagement with the one tool to an engagement with the second tool. In certain embodiments of connectors and tools, both engagements are frictional, and are disengaged by pulling a tool away from the connector. With these systems, a force required to dis-engage (the "force of engagement") one tool can preferably be lower than the force required to dis-engage the other tool from the connector. For example, a force of engagement between an insertion tool and a connector can be lower than a force of engagement between a removal tool and a connector. This can allow the connector to be passed (transferred) from the insertion tool to the removal tool by engaging the connector with the insertion tool, placing the connector internally to the patient, engaging the connector and removal tool, then dis-engaging the insertion tool.

A connector can be a structure that can attach (directly or indirectly) to an extension portion, e.g., at or near a distal end, and that can also engage two tools, preferably at the same time, for manipulation of the connector by and between the tools. An example of a connector can be a plastic (alternately metal) tip located at or near a distal end of an end portion of an implant, having two ends, a longitudinal axis that extends between the two ends, and sides that extend between the two ends. The two ends can for convenience be referred to as a proximal end and a distal end, but these designations are not required and should not be considered to limit the structural features of a connector as described. A distal end can generally be considered to be an end located distal relative to the tissue support portion, and the proximal end can be considered to be an end located more proximal to the tissue support portion. Regardless of these potential designations, each of the two ends engages a tool that allows manipulation of the connector by the tool. The engagement may be any functional engagement that can be frictional, removable, secure, permanent, etc., and that can be achieved by contacting an end (e.g., tip) of a tool with an end of the connector. The engagement may involve friction, snapping or snap-fitting, threading, locking, a detent, a spring, a deflected surface, or any other mode of engaging a connector and tool.

Optional features of a connector can relate to shape and size. A connector can be any useful shape and size. An exemplary connector may be substantially cylindrical, optionally including a bend or a curve. A connector may be a "dilating" connector that exhibits a variable size (e.g., diameter) along a length extending from the proximal end to the distal end, such as a narrowed or tapered distal end, to assist in passing the connector through tissue.

Dimensions of a connector can be any that allow engagement and manipulation of the connector by two different tools, e.g., at two different ends, preferably simultaneously to allow the connector to be useful according to exemplary methods as described. A length can preferably be sufficient to allow the connector to engage two different tools separately or simultaneously, at opposite ends, with good control of the connector for manipulation of the connector by manipulation of each of the tools together or independently. An exemplary range of useful length between ends of a connector may be from about 0.5 to about 4 centimeters, such as from 0.7 to 3 centimeters or from 0.7 to 2 centimeters. Exemplary cross-sectional dimensions, e.g., width or diameter, may be smaller than the length, and may be sufficient to allow the connector to engage the tools and also allow the connector to pass through tissue (e.g., muscle or ligament) of pelvic region. Exemplary widths or diameters may be from about 1 or 2 millimeters to about 7 millimeters, e.g., from 2 to 5 or 6 millimeters.

A connector can be made out of any useful material, generally including polymeric materials that can be molded or formed to a desired structure and connected or attached, directly or indirectly, at a distal end of an extension portion of an implant. Useful materials can include plastics such as polyethylene, polypropylene, and other thermoplastic or thermoformable materials, as well as metals or ceramics if desired.

An end of a connector (e.g., a proximal end) can be designed to engage the distal end of a tool (e.g., a tip of an insertion tool). The engagement can be a removable (e.g., non-permanent) engagement, meaning that the distal end of the tool can be engaged with and easily dis-engaged from the connector. As desired, the engagement may be a mechanical or frictional engagement that allows an end of the tool to engage the connector. Examples include a snap-fit engagement; a mechanical grasping engagement that can be controlled (e.g., engaged and disengaged) by a grasping mechanism at a proximal end of the tool; a shaped engagement such as a hook; a threaded engagement; an engagement that includes a detent mechanism or a spring, or a simple engagement between two smooth surfaces such as a cylindrical tip and a cylindrical bore.

Functionally, an engagement between an insertion tool and a connector can be sufficient to allow the insertion tool to insert the connector through an incision, into a pelvic region. When inserting a connector into a patient's pelvic region, the connector does not experience a relatively high amount of resistance or friction, especially if the insertion is through a vaginal incision. Depending on the nature of the incision and the desired placement of a connector, the engagement may required to generally push the connector through an incision and to a space internal to the pelvic region, which would not require a substantially secure engagement but only a relatively low or even minimal frictional engagement. The engagement may need to be somewhat stronger if the insertion tool is designed to pulls or drags the connector into the pelvic region.

An example of a removable engagement can be a frictional engagement between two smooth surfaces, one smooth surface of a distal end (e.g., tip) of an insertion tool, and one smooth surface of an aperture extending from an end of a connector. For example, a tip of an insertion tool may include a cylindrical end that engages a bore at a proximal end of the connector. The opposing surfaces of the insertion tool tip and the bore of the connector may be smooth, without any locking, threading, or snap-fit mechanism. Optionally or alternately, a curved, cornered, or angled surface can be included in the bore, corresponding to a complementary surface of the insertion tool, such as a keyed surface, if desired.

An end of a connector (e.g., a distal end) can be designed to engage a distal end of a second tool (e.g., a removal tool). The removal tool can preferably engage the connector in a manner that allows the removal tool to pull the connector through a tissue path without the removal tool becoming dis-engaged from the connector; e.g., the engagement may be a "secure" engagement. A "secure" engagement is an engagement that allows an end of the removal tool to engage the connector in a manner by which the removal tool can pull the connector through tissue, without the connector becoming dis-engaged from the removal tool. Exemplary secure engagements may include a snap-fit engagement; a mechanical grasping engagement that can be controlled (e.g., engaged and disengaged) by a grasping mechanism at a proximal end of the tool; a shaped engagement such as a hook; a frictional sliding engagement that includes a secure snap-fit, locking, or threaded engagement. A secure engagement can preferably be a permanent engagement. A "permanent engagement" is an engagement that would be very difficult to manually separate, once an engagement is made, such as can be true of a snap-fit engagement.

A tissue path through which a connector might be pulled, using a removal tool, may pass though tissue including one or more of muscle tissue, ligament tissue, skin, and the like, such as a tissue path that extends from a patient's pelvic region interior, through tissue of the pelvic floor, coccygeus muscle, sacrospinous ligament, gluteus muscle, abdomen, perineum, or obturator foramen, and then optionally and preferably through an external incision. A secure engagement between a removal tool and a connector can be sufficiently strong to prevent disengagement between the removal tool and the connector, when using the removal tool to pull the connector through such a tissue path.

An insertion tool can be a tool that includes a shaft, optional handle, and a distal end (including a "tip") that engages a connector. The handle and shaft can be relatively straight and arranged along a single longitudinal axis. Alternately, a handle or shaft may be bent or slightly curved. A handle may be distinct from a shaft, or a handle and shaft may converge or taper together, e.g., because these can lie along a single axis and can optionally and preferably be of a single, integral, one-piece molded construction.

A distal end of a shaft can engage a connector by any mode, such as by a moveable mechanical engagement (e.g., jaws similar to a pliers), by a moveable mechanical detent that corresponds to a surface of the corinector, by a threaded engagement, a snap-fit engagement, by a smooth surface engagement between a smooth cylindrical tip (optionally bent or curved) that engages a smooth bore of a connector, or by any other mechanical or frictional engagement.

An exemplary embodiment of a tip can be an elongate cylindrical tip that engages an elongate bore of a connector. The elongate cylindrical tip can be of any useful length and width dimensions. An exemplary length of an elongate cylinder tip, e.g., measured as a straight portion of a tip (not including a bend portion of an insertion tool distal end) can be in the range from 2 to 6 millimeters, e.g., from 3 to 5 millimeters. An exemplary width dimension (e.g., diameter) can be from 1 to 6 millimeters, e.g., from 2 to 5 millimeters.

According to certain embodiments of tools and connectors, a distal end of an insertion tool includes a bend at an end of a shaft, prior to a cylindrical tip. A bend at the end of the shaft can allow the tool to place the connector into a pelvic region while the connector is held in an angled orientation relative to the shaft of the insertion tool. The bend can place an elongate tip at any desired angle relative to the shaft of the insertion tool. By way of example, an angle between a shaft and an elongate tip may be in the range between an acute angle of about 45 degrees relative to the shaft, to no angle (e.g., a straight shaft), or an obtuse angle such as 170 degrees, e.g., 135 degrees relative to the shaft. If the angle is an obtuse angle of, e.g., greater than 165 degrees, resulting in an insertion tool that pulls or drags the connector into the pelvic region, the force of engagement can be made stronger, such as by a loose snap-fit configuration. The bend between a shaft and an elongate tip is preferably relatively sharp, such as to define a radius of curvature that is less than 2 centimeters, such as less than 1.5 centimeter, e.g., from 1.5 centimeter to 0.5 centimeter.

An embodiment of an insertion tool can include a shaft, a handle on a proximal end of the shaft, and a single distal end that engages a connector. An alternate embodiment of insertion tool can include a shaft, optionally a handle that is central (medial) to the tool between two opposing shafts, and two ends (eg., two distal ends) extending from two opposing shafts at opposite ends of the handle. Each end can be configured to engage a connector, and each end may be capable of engaging the same or similar connector, or different connectors. Each end may include an elongate cylindrical tip that may be angled from the shaft. Each angle may be a different angle, each angle being useful to accommodate placement and orientation of a connector differently within a pelvic region, with each orientation allowing an end of a connector to engage a second (e.g., removal) tool that approaches the connector from a location within the pelvic region.

For example, an end of an insertion tool may include an elongate cylinder tip that is angled relative to the shaft to allow placement of a connector at a posterior pelvic location (e.g., at a rectovaginal or pararectal space, such as near a sacrospinous ligament, ischial spine, coccygeus muscle, coccyx bone, sacrum, etc.), at an orientation (angle) that points the opposite end of the connector in a direction that allows the opposite end to engage a second (e.g., removal) tool that approaches the connector from a posterior pelvic location (e.g., from a location at a sacrospinous ligament, ischial spine, or coccygeus muscle). The angle may be an obtuse angle that allows the insertion tool to engage a connector, insert the connector into a vaginal incision, then move (e.g., push from behind) the connector in a posterior or lateral direction toward posterior or lateral tissue such as tissue of an a sacrospinous. ligament, ischial spine, coccygeus muscle, etc., to engage a removal tool that approaches the connector from that location; exemplary angles may be in the range of an obtuse angle, such as from 90 to 170 degrees.

An end of an insertion tool includes an elongate cylinder tip that is angled relative to the shaft to allow the tool to place a connector at a location within the pelvic region, at a location and orientation (e.g., angle) that allows the opposite end of the connector to be aligned to engage a second (removal) tool that is approaches the connector from an anterior pelvic location (e.g., from tissue of an arcus tendineus, pubic symphasis, or obturator foramen). The angle may be an acute angle that allows the insertion tool to engage a connector, insert the connector into a vaginal incision with the connector oriented to receive a removal tool that approaches the connector from an anterior location; exemplary angles may be in the range of an acute angle, such as from 75 to 90 degrees, e.g., from 70 to 85 degree.

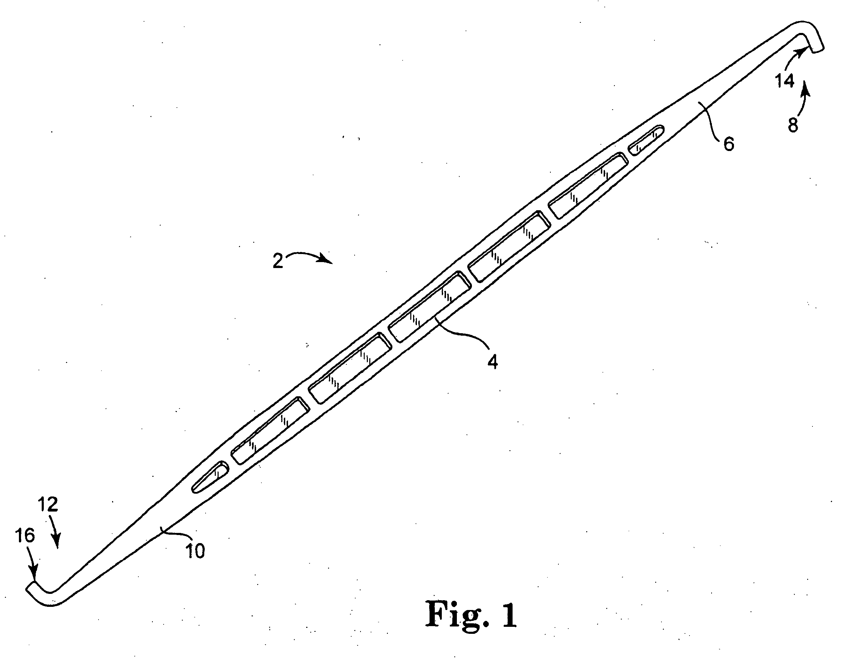

An insertion tool can be made of any materials, such as plastic or metal. A shaft and distal end may be metal, and the shaft may be attached to a plastic handle. Alternately, and preferably, due to a potentially simple design (e.g., as shown at figures 1, 2, and 3), an insertion tool can be prepared as a one-piece integral corlstruction from metal or polymeric material, such as a moldable plastic. Particularly preferred embodiments of insertion tools can be of a one-piece construction, made of an integral piece of plastic or metal that has been molded or otherwise formed to include an integral handle, shaft, and tip, from a single, integral piece of material. An example of this type of integral tool may be made by injection molding of plastic. Such a tool can be relatively straight, with straight handle and one or more straight shafts aligned along a single axis, and one or two angled tips at on end or at two opposite ends of the tool.

A removal tool can be used in conjunction with the insertion tool to install the implant. Various types of tools are known for use in placing pelvic implants. A removal tool as described herein can be a tool that engages an end of a connector in a manner that allows the removal tool to pass the connector through tissue, preferably to pull the connector through a tissue path in the pelvic region. A tissue path may be between tissues internal to a pelvic region, but typically can be a tissue path between a location internal to the pelvic region, through tissue of a pelvic region (e.g., muscle, ligament, fascia, etc.), to an external-incision.

Examples of useful tools include those types of tools that generally include a thin elongate needle (or shaft) that attaches to a handle; a handle attached to one end (a proximal end) of the needle; and a distal end of the needle adapted to engage a connector in a manner that allows the needle to pull the connector through a tissue path, e.g., through a secure engagement or optionally a permanent engagement.

A useful removal tool can be designed, shaped, and sized, to include an elongate "needle" or "shaft" that may be straight or that may be curved in two or three dimensions, and can preferably be inserted through an incision and through a tissue path that leads to a location of a connector (at an end of an insertion tool) within the pelvic region. The incision may be, for example, a vaginal incision (for female anatomy), a perineal incision (for female or male anatomy), an incision in the rectal or buttock region, at the inner thigh or groin (for a transobturator tissue path), or at an abdominal region, etc. A two-dimensionally curved needle may, for example, extend from a perirectal region, through a tissue path in a direction toward an ischial spine, through tissue of the sacrospinous ligament, coccygeus muscle, or both, then to a region of the vagina. A helical needle may extend from an incision at the inner thigh, through an obturator foramen and tissue of the obturator foramen, then to a region of a urethra or a vaginal incision (for a female), or a region of a urethra and a perineal incision (for a male).

In general, a removal tool can include a handle, needle (or shaft), needle distal end, and needle tip. The handle is at the portion of the tool that will be referred to as a proximal end, and the needle tip is at the needle distal end. A proximal end of the needle connects to a distal end of the handle. The "needle tip" refers to a length of a needle that is at the needle distal end, including a portion that engages a connector.

Exemplary insertion tools for treatment of pelvic conditions are described, e.g., in United States patent number 7,070,556; United States patent number 6,802,807; United States patent number 6,641,525; United States patent number 7,037,255; United States patent number 6,911,003; United States patent number 7,070,556; United States patent publication numbers 2005/0245787, 2006/0235262, 2006/0260618, and 2005/0250977; and International patent application numbers PCT/US2006/028828 and PCT/2007/016760, now published as WO 2007/016083 and WO 2008/013867 respectively, among others. For use according to methods described herein, those or similar tools may be designed with a distal end or tip that engages a connector as described herein.

Figure 1 shows an example of an insertion tool. Tool 2 includes handle 4, shaft 6, distal end 8, shaft 10, and distal end 12. Distal ends 8 and 12 include tips 14 and 16, which are elongate cylindrical tips, and each of which is bent relative to shafts 6 and 10. Each of tips 14 and 16 is shaped to engage a bore of a connector. The angle between tip 14 and shaft 6 is acute, e.g., is from about 80 to 90 degrees relative to the longitudinal axis of shaft 6. The angle between tip 16 and shaft 20 is not acute, but is a "mid angle," e.g., is from 90 to about 110 degrees relative to the longitudinal axis of shaft 10. The total length of the tool can be, e.g., from about 15 to 30 centimeters, e.g., from 22 to 26 centimeters. The length of the medial handle can be about 11 to 17 centimeters, e.g., from about 12 to 16 centimeters. Each shaft is illustrated to be tapered back to the handle but may be non-tapered. Each shaft is from about 2 to 6 centimeters in length, e.g., from about 3 to 5 centimeters long

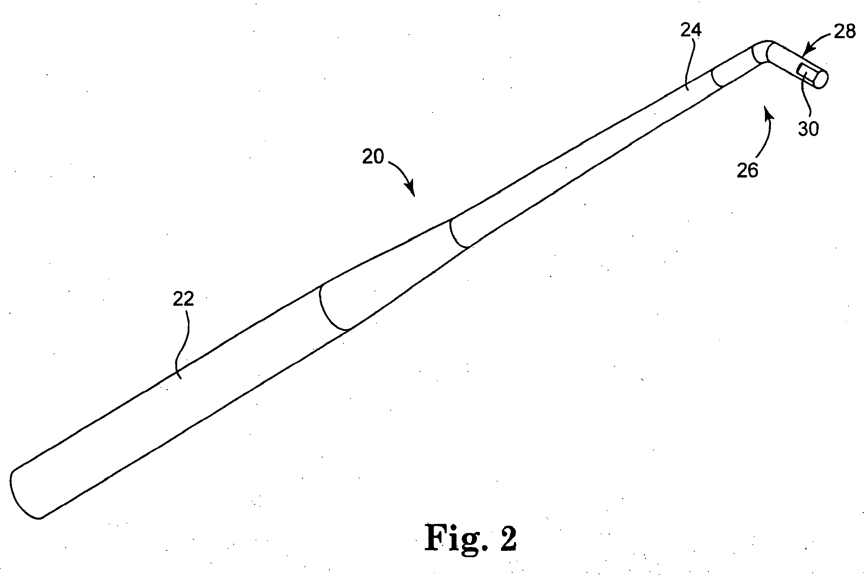

Figure 2 shows another example of an insertion tool. Tool 20 includes handle 22, shaft 24, distal end 26, and tip 28. This embodiment only includes a single distal end, 26, that includes a tip (28) for engaging a connector. The angle between tip 28 and shaft 24 is obtuse, e.g., can be from about 90 to about 175 degrees (or may be straight); as illustrated the angle is in a range from about 100 to 140 degrees, e.g., from about 110 to 130 degrees, relative to the longitudinal axis of shaft 24. Tip 28 is an elongate cylindrical tip shaped to engage a bore of a connector, and includes flat surface 30, which corresponds to a flat surface of the bore, to require alignment of the connector to tip 28 upon engagement.

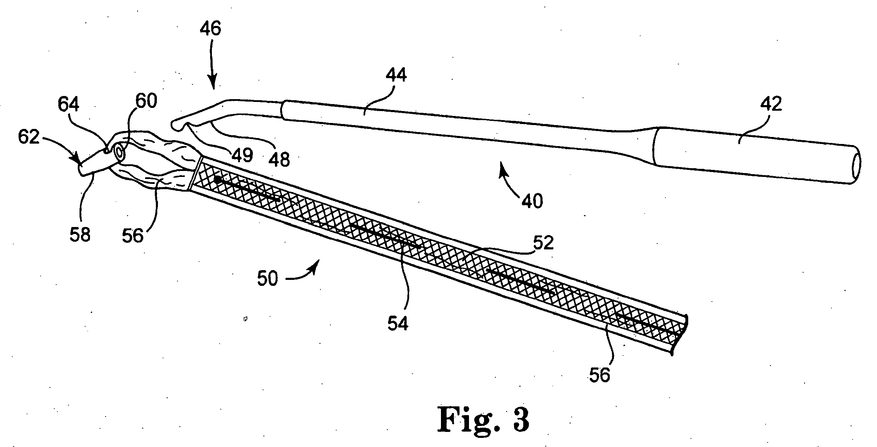

Figure 3 shows another example of an insertion tool, tool 40, in combination with implant 50. Tool 40 includes handle 42, shaft 44, distal end 46, and tip 48. The angle between tip 48 and shaft 44 is obtuse; as illustrated the angle is in a range from about 150 to 165 degrees relative to the longitudinal axis of shaft 44. Tip 48 engages a cylindrical bore at proximal end 60 of connector 58 of implant 50. Tip 48 also includes recessed surface 48, which can allow for a stronger engagement between connector 58 and tip 48, such as by a snap-fit or detent mechanism. Connector 58 also includes distal end 62, which is designed to engage a tip of a removal tool. Implant 50 generally includes mesh extension portion 52, including tensioning suture 54, and which is covered by sheath 56. Sheath 56 extends past the distal end of mesh 52 to attach to connector 58, through bore 64, which extends laterally from one side of connector 58 to another side, between distal end 62 and proximal end 60.

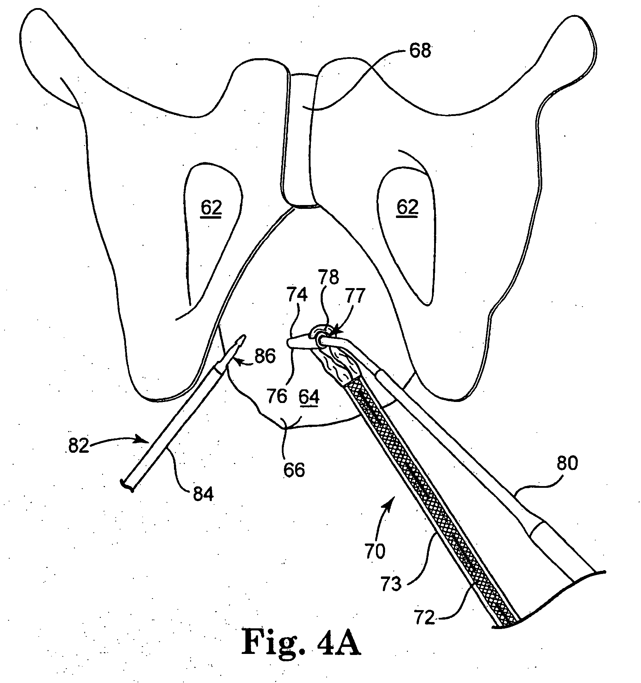

Figure 4A illustrates a step of inserting an implant through a vaginal incision, using an insertion tool, and placing a connector of the implant at a location to engage a tip of a removal tool. Relevant anatomy includes opposing obturator foramen 62, sacrum 64, coccyx bone 66, and pubic symphasis 68. Referring to the figure, implant 70 includes extension portion 72 surrounded by plastic sheath 73, which is attached to connector 74. Connector 74 includes distal end 76 and proximal end 78. Proximal end 78 is engaged with a tip of insertion tool 80. As illustrated, a distal end of insertion tool 80, which includes tip 77, is engaged with proximal end 78 of connector 74, and extends through a vaginal incision (not shown). Connector 74 is located at a posterior location within the pelvic region of a patient, e:g., posterior to vaginal tissue, near one or more of a rectum, sacrospinous ligament, rectovaginal space, pararectal space, etc. The angle of tip 77 relative to the shaft of insertion tool 80 causes connector 74 to be oriented with distal end 76 pointing in a direction of removal tool 82. Removal tool 82 includes a needle (e.g., curved) 84 and tip 86. Needle 84 is inserted from an external incision (e.g., in a perirectal region of a patient), through tissue leading to the posterior location of the pelvic region, e.g., through coccygeus muscle, sacrospinous ligament, gluteus muscle, or the like. Tip 86 is placed at a posterior location of the pelvic region to engage distal end 76 of connector 74.

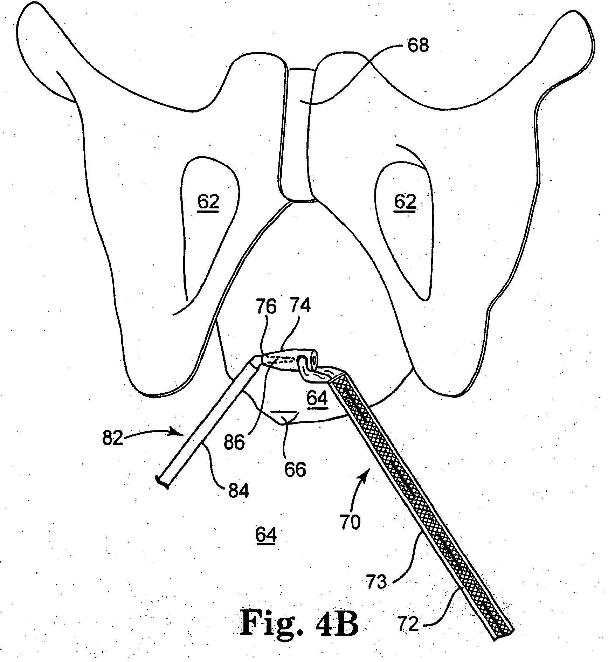

Referring to figure 4B, distal end 76 of connector 74 is engaged with tip 86 (shown in shadow) of removal tool 82, by pushing tip 86 into a bore extending from distal end 76 into connector 74. The engagement between tip 86 and connector 74 is preferably a secure engagement, such as a secure or permanent snap-fit engagement, and also preferably exhibits a stronger force of engagement than the force of engagement between tip 77 and connector 74. This difference between the forces of engagement, allows insertion tool 80 to be removed from engagement with connector 74, with connector 74 remaining engaged to tip 86 of removal tool 82. Removal tool 82 can then be retracted to pull connector 74 through the tissue path leading to the perirectal region. Extension portion 72 and sheath 73 can be manipulated and adjusted. Sheath 73 can be cut and removed to place extension portion 72 in the tissue path as desired.

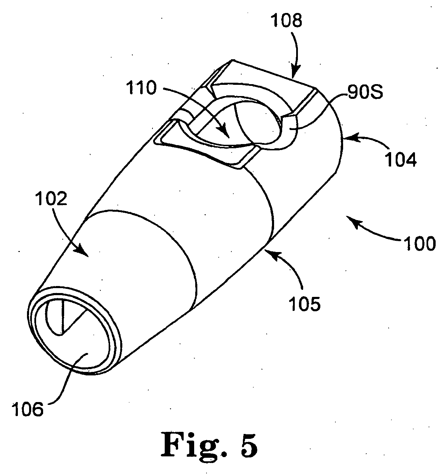

Figure 5 shows a perspective view of an embodiment of a dilator useful according to the present description. Dilator 100 includes distal end 102, proximal end 104, and sides 105 (cylindrical) between the distal and the proximal ends. Aperture 110 is located at a side, and an opposing aperture (not shown) is located on a side opposite of aperture 110. Bore 106 is at distal end 102, and is sized and configured to securely engage a tip of a removal tool. Bore 108 is at proximal end 104 and is sized and configured to removably engage a tip of an insertion tool Aperture 110 and the aperture (not shown) that is opposite of aperture 110 together provide a bore that is lateral to a longitudinal axis of connector 100, through which a sheath or other element of an implant can be placed to attach the connector to the sheath or other element.

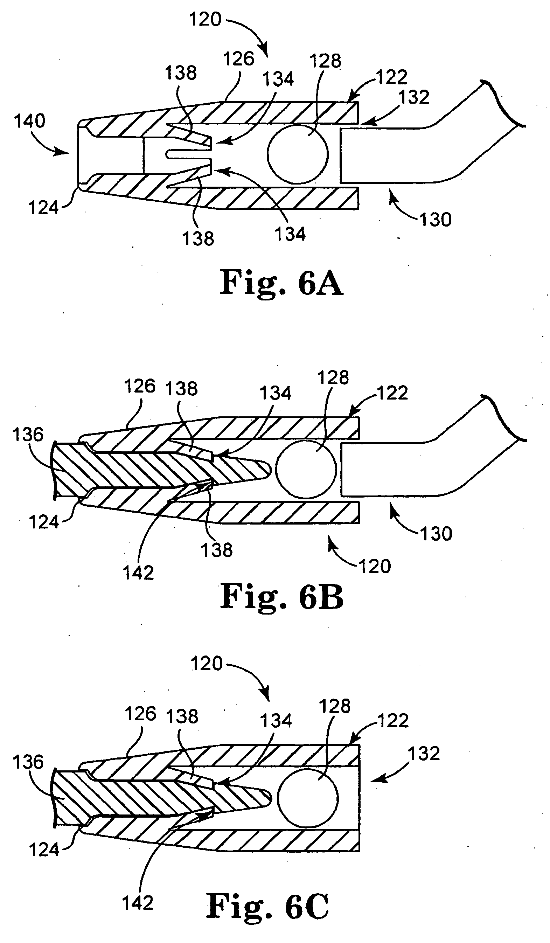

Figures 6A, 6B, and 6C illustrate a sequence of removably engaging an insertion tool with an end of a connector, and transferring the connector from the removable engagement with the insertion tool, to a secure (and permanent) engagement with a removal tool. Referring to figure 6A (a cross-section), connector 120 includes proximal end 122, distal end 124, sides 126 extending between proximal end 122 and distal end 124, and bore 128 extending laterally through connector 120 from side to side. Proximal end 122 includes bore 132 for receiving tip 130 of an insertion tool. Distal end 124 includes bore 140 for receiving a tip of a removal tool (not shown). Tip 130 of the insertion tool removably engages the internal surface of bore 132 by a snug fit between smooth surfaces of tip 130 and bore 132. The removable engagement is sufficient to allow connector 120 to be retained at tip 130 during placement of connector 120 at a position internal to a patient, e.g., through a vaginal incision. The removable engagement can then become disengaged as desired by use of a removal tool inserted into bore 140, to pull connector 120 from tip 130.

Figure 6A shows internal stop surfaces 134, which are complementary with specially shaped surfaces on a tip of a removal tool. Each stop surface 134 is designed to engage complementary shoulder surface of a tip of a removal tool to achieve a snap-fit, secure and permanent engagement. Figure 6B (also in cross section) shows the features of figure 6A and additionally shows tip 136 of a removal tool that has been inserted into aperture 140, that snap-fits within the aperture and deflecting surfaces 138. Shoulder 142 of tip 136 engages stop surfaces 134 to provide a secure, one-way snap fit that is permanent in that a user would have difficulty manually removing tip 136 from connector 120.

Figure 6C (also in cross section) illustrates connector 120 engaged with tip 136 upon removal of tip 130. The engagement shown at figure 6C allows a user to remove connector 120 from within a pelvic region of a patient by removing the removal tool; tip 136 of the removal tool is securely engaged with connector 120, so as the removal tool is pulled back through a tissue path, through tissue (e.g., to an external incision), connector 120 remains engaged. An implant attached to connector 120 is pulled through the tissue path. The implant can be connected in any manner, such as by a sheath threaded through aperture 128. After placement of the implant, to separate the implant from the connector, the user cuts an end of the sheath (and optionally implant) near connector 120 to separate the implant from the connector and engaged tip 136 of a removal tool.

In use, the engagement between tip 136 and connector 120 preferably affords quick and convenient attachment of the connector 120 to tip 136, as connector 120 is engaged with and manipulated by an insertion tool having tip 130. The engagement between tip 130 and connector 120 has a force of engagement that is less than the force of engagement between tip 136 and connector 120, to allow connector 120 to be transferred from the engagement with tip 130 to the engagement with tip 136. The engagement between tip 136 and connector 120 is secure, to allow the engagement to be maintained while tip 136 pulls connector 120 through tissue.

The implants and tools described herein can be used to treat pelvic conditions by implanting the implant using two tools as described, with the tools being used to manipulate the connector. Tools and implants as described can be useful for treating conditions of the pelvic region such as any one or more of incontinence (various forms such as fecal incontinence, stress urinary incontinence, urge incontinence, mixed incontinence, etc.), vaginal prolapse (including various forms such as enterocele, cystocele, rectocele, apical or vault prolapse, uterine descent, etc.), conditions of the perineal body, conditions of the pelvic floor including the coccygeous and levator muscle (such as by treating a component of levator muscle, iliococcygeous, coccygeous, levator ani, pubococcygeous), conditions of the levator hiatus, and combinations of two or more of these, and other pelvic conditions caused by muscle and ligament weakness.

According to certain exemplary methods of treatment, a surgical implant can be used to treat a pelvic condition, wherein the method includes placing an implant in a manner to support tissue of the pelvic region in a male or female. The tissue may be tissue of a bladder, bladder neck, urethra, vagina, rectum, etc. The method can involve an implant that includes a connector as described and two surgical tools, each of which engages the connector, and can both engage the same connector at the same time to allow the connector to be transferred from engaging one tool, to engaging the other tool, at a location internal to the patient. Exemplary tools can include one tool (e.g., an insertion tool) that engages one end of the connector and a second tool (e.g., a removal tool) that engages another end of the connector. For example, an insertion tool may engage the connector, and the insertion tool can be used to pass the connector through an incision into the pelvic region; the incision may be an external incision such as an abdominal or perineal incision, or a vaginal incision. A distal end of a removal tool is passed through a different incision, e.g., an external incision, to locate the distal end (e.g., tip) of the removal tool at a location internal to the patient. The distal end of the removal tool engages the connector, already placed at a location internal to the patient using the insertion tool. The insertion tool can be disengaged from the connector and removed. The removal tool, now in engagement with the connector, is used to remove the connector from the patient and preferably to guide an extension portion of the implant into a desired tissue path; during the step of removing the removal tool, an extension portion of the implant attached (directly or indirectly) to the implant becomes placed in the tissue path.

General steps of exemplary methods can include an initial step of engaging an insertion tool with a connector. This may be done at a location that is either internal to or external to a patient, preferably external to the patient. The insertion tool can be used to place the engaged connector into a patient. For treating female urinary incontinence, the insertion tool is passed through a vaginal incision. Other incisions can be used for treating other conditions. Another step can be to insert a removal tool through an external incision, to place a distal end of the removal tool at a location for the distal end to engage the connector (engaged with the insertion tool). As examples, a removal tool may be passed through a perirectal incision, a perineal incision, or through an incision at the inner thigh, to either create or pass through a tissue path into which an extension portion of the implant will become placed. Internal to the patient, the removal tool is engaged with the connector. The removal tool is then withdrawn from the patient to remove the connector and to bring a portion of the implant (e.g., an extension portion) into the tissue path used by the removal tool.

Particular exemplary methods can generally involve an implant that includes a connector as described, an insertion tool, and a removal tool, used to place a surgical implant in a manner that support pelvic tissue. Placement of the implant can be by any incision and dissection route, with particular methods involving one or more of a vaginal incision, perineal incision, a perirectal incision, or a perianal incision.

Features of useful exemplary methods include features of methods for treating female urinary incontinence as described in United States Patent 6,911,003 (serial number 10/377,101, filed March 3, 2003), and United States Patent Application Publication 2003/0171644 (serial number 10/306,179, filed November 27, 2002). Methods described in those patent documents can be modified to include the use of tools and a connector, as described. As modified, exemplary steps of such a method may include placement of a urethral sling via a transobturator passage created using a removal tool, and placement of an implant through a vaginal incision using an insertion tool. A small incision (e.g. a transverse incision) is made in the anterior vaginal wall of a patient followed by a transurethral dissection. The amount of dissection may vary according to surgeon preference, and may be reduced or minimized by use of an insertion tool as described herein. Two small stab incisions are made near the obturator fascia to afford entry of a needle portion of a removal tool.

A distal end and tip of a removal tool (e.g. having a helical needle) is passed through a stab incision on one side of the patient, and is further passed through the obturator fascia and the obturator foramen to place the distal end of the needle near the vaginal tissue, such as near the vaginal incision. The connector is engaged with the end of the insertion tool, and the insertion tool is used to transvaginally place the connector through the vaginal incision. The connector is placed in a position to allow the distal end of the removal tool to engage the connector. The removal tool is engaged with the connector, and the insertion tool is dis-engaged with the connector. The insertion tool is retracted from the vaginal incision. The removal tool is retracted from the trans-obturator tissue path, pulling the connector to a location external to the patient, and pulling an extension portion of the implant into the tissue path created by the removal tool. The implant is adjusted to support the urethra. The steps can be repeated on the other side of the patient.

As opposed to previous methods this procedure can require less intrusion (e.g., dissection) into the patient's pelvic region due to the use of the insertion tool instead of a surgeon's fingers or hands. The tip of the removal tool needle is not required to be passed through the vaginal incision to allow connection of the connector to the helical removal tool needle, and the surgeon is not required to use his or her fingers to connect the connector to the helical removal tool needle at a location internal to the patient.

Exemplary methods can also be used to treat f male urinary incontinence, such as by the exemplary method that includes features described in Assignee's copending United States Patent Application Publication US 2006 287571 A1 filed February 3,2006 ("Transobturator Methods for Installing Sling to Treat Incontinence, and Related Devices"). Such a method can begin by making a pair of lateral incisions substantially adjacent a patient's left and right obturator foramen. A medial incision is made at the perineum to expose bulbospongiosus muscle. The bulbospongiosus muscle is dissected to expose corpus spongiosum. A needle of a removal tool is passed through a lateral incision, through the obturator foramen, and the distal end is placed at a region near the medial incision. A connector is engaged with a distal end of an insertion tool and the insertion tool is used to pass the connector through the medial incision to a location near the distal end of the removal tool. The connector engages the removal tool, the insertion tool is disengaged from the connector and removed. The removal tool is retracted from the trans-obturator tissue path, pulling the connector to a location external to the patient, and pulling an extension portion of the implant into the tissue path created by the removal tool. The implant is adjusted to support the urethra. A support portion of an implant is placed to contact the corpus spongiosum. The steps can be repeated on the other side of the patient.

Tools and implants can be useful to treat pelvic organ prolapse, such as posterior vaginal prolapse, rectocele, vault prolapse, etc., using a posterior tissue path passing from a location of posterior vaginal tissue to one or more external incisions in a perirectal region. The exemplary methods may include features of methods described in United States Patent Application Publication 2005/0245787 (serial number 10/834,343) filed April 30,2004 ("Method and Apparatus for Treating Pelvic Organ Prolapse"). Such a method can begin by accessing the external vaginal vault using any method of incision and dissection, including a vaginal incision. Two small stab incisions are made on each side of the rectum approximately 3 cm lateral and 3 cm posterior to the anus. A needle of a removal tool is pointed perpendicular to the skin with the handle pointing upward in a 12:00 position, and the needle is directed at a slight upward and lateral angle through the stab incision and buttock. The needle punctures the initial layers of tissue by the surgeon pushing on the needle until the needle enters the ischiorectal fossa. The surgeon continues to pass the needle tip lateral and parallel to the rectum, toward the ischial spine, with palpation as needed. The removal tool needle tip can be palpated in front of the ischial spine and can be advanced to penetrate the levator muscle, advancing and lightly turning the needle tip medially toward the vaginal vault. Separately, e.g., external to the patient, a connector of an implant is engaged with a distal end of an insertion tool. The insertion tool is used to pass the connector through the vaginal incision and into engagement with the distal end of the removal tool located near the vaginal vault. The insertion tool is disengaged from the connector and removed from the vaginal incision. The needle of the removal tool is used to pull the connector back through the skin incision, placing an extension portion of the implant in the tissue path created by and used by the removal tool. The implant is attached to the exterior apex of the vaginal wall (e.g., with two or more sutures) and the implant is adjusted. The steps can be repeated on the other side of the patient through the second stab incision.

Tools and implants can be useful for treating other types of vaginal prolapse, such as anterior vaginal prolapse, using an anterior tissue path passing from a location of anterior vaginal tissue to one or more external incision in a region of the inner thigh or groin, the tissue path passing through an obturator foramen. The exemplary method can include features as described in United States Patent Application Publication 2005/0250977 (serial number 10/840,646) filed May 7, 2004 ("Method and Apparatus for Cystocele Repair"). Such a method can begin by making a vaginal incision starting below the bladder neck, over the most prominent part of the prolapse, to the lowermost part of the prolapse. The patient's bladder is dissected off the vagina up to the lateral sulcus and posterior to the vaginal vault. This dissection allows palpation of the medial edge of the inferior pubic ramus, assisting in guiding superior and inferior needles of removal tools. The patient's cystocele is then reduced using midline plication, if desired. Next, markings are made to identify the locations for entry incisions for needles of removal tools. The vaginal dissection is completed prior to marking needle entry incisions to allow for digital palpation along the ischiopubic ramus. The needle entry points are palpated internally and externally with the thumb and index finger before marking, as discussed hereafter. The edge of the ischiopubic ramus beginning at the level of the vaginal incision is palpated, continuing along the edge of the bone cephalad toward the level of the clitoris denoting where the adductor longus tendon inserts into the pubic ramus. Two superior skin incisions are marked approximately at this location and lateral to the edge of the bone. The markings are made according to the same method on both sides (right and left) of the patient's body. Both marks lie in a straight line at the approximate level of the clitoris. The edge of the inferior pubic ramus is palpated until it ends at the bottom of the obturator foramen. Two inferior skin incisions are then marked. The inferior skin incisions are located at a point approximately 3 centimeters below and 2 centimeters lateral to the superior marks. The markings are made according to the same method on both sides of the patient's body.

A small vertical stab incision is made over all four markings to provide needle entry incisions for needles of removal tools. A tip of a superior removal tool needle is inserted through the left superior incision, through the left obturator foramen, and to a region of the vaginal incision. Separately, e.g., external to the patient, a connector of an implant is engaged with a distal end of an insertion tool. The insertion tool is used to pass the connector through the vaginal incision and into engagement with the distal end of the removal tool needle located near the vaginal incision. The insertion tool is disengaged from the connector and removed from the vaginal incision. The needle of the removal tool is used to pull the connector back through the skin incision, placing an extension portion of the implant in the tissue path used by the removal tool, passing through an obturator foramen. The implant is attached to vaginal issue.

Additional steps can be used to pass additional extension portions of the implant through the three other external incisions. Four external incisions are described, but fewer can be used. Also, alternately or in addition, extension portions of an implant may be passed through posterior tissue paths, e.g., leading to external incisions in a perirectal region.

## Claims

1. In combination, a pelvic implant (50;70), an insertion tool (2;20;40;80), and a removal tool (82),
the implant (50;70) comprising
a tissue support portion.
an elongate extension portion (52;72) having a proximal end attached to the tissue support portion and a distal end, and
a connector (58;74;100;120) associated with the distal end, the connector (58;74;100;120) comprising a connector proximal end (60;78;122) and a connector distal end (62;76;124);
the insertion tool (2;20;40;80) comprising an elongate shaft (6;10;24;44) having a distal end configured to engage the proximal end (60; 78; 122) of the connector (58; 74; 100; 120) and
the removal tool (82) comprising a distal end (86;136) configured to engage the distal end (62;76;124) of the connector (58;74;100;120);
**characterised in that** the elongate shaft (6,10;24;44) of the insertion tool (2;20;40;80) has a distal end comprising a straight portion and angled elongate tip, the angled elongate tip being configured to engage the proximal end of the connector as the connector is at an angled orientation relative to the straight portion.

2. A combination according to claim 1 wherein
the connector (58;74;100;120) comprises an elongate cylinder (105;126) comprising an elongate axis extending along a length of the connector (58;74;100;120), a proximal aperture (132) extending a portion of the length from the connector proximal end (60;78;122), and a distal aperture (140) extending a portion of the length from the connector distal end (62;76;124);
wherein the insertion tool (2;20;40;80) comprises a tip (130) that removably engages the proximal aperture (132) and the removal tool (82) comprises a tip (86;136) that securely engages the distal aperture (140).

3. A combination according to claim 2 wherein the tip (86;136) of the removal tool (82) engages the distal aperture (140) in a permanent snap-fit arrangement.

4. A combination according to any of claims 1 to 3 wherein the connector (58;74;100;120) is a dilating connector (100) comprising a tapered distal end (102).

5. A combination according to any of claims 2 through 4 wherein
the connector (58;74;100;120) comprises sides (105) extending from the connector proximal end (104) to the connector distal end (102), and further comprises a bore (106) extending through the connector (100) from one side to another side, and
the implant (50;70) comprises a sheath (56;73) covering at least a portion of the extension portion, the sheath (56;73) extending through the bore (106) and forming a loop to attach the sheath (56;73) to the connector (58; 74; 100; 120).

6. A combination according to any of claims 1 through 5 wherein the removal tool (82) comprising an elongate needle and a distal end (86;136) configured to permanently engage a distal end (62;76;124) of the connector (58;74;100;120).

7. A combination according to any of claims 1 through 6 wherein the insertion tool (2) comprises two opposing shafts (6,10) each having an end (8,12);
a first cylindrical tip (14) at the first shaft end (8); and
a second cylindrical tip (16) at the second shaft end (12).

8. A combination according to claim 7 wherein the first tip (14) is at an angle relative to the first shaft end (8) and the second tip (16) is angled relative to the second shaft end (12), and each angle is different.

9. A combination according to claim 8 wherein one angle is an acute angle and one angle is an obtuse angle.

10. A combination according to claim 1 wherein the insertion tool (2;20) comprises a handle (4;22;42), a shaft (6;10;20;44) that includes a straight portion, and a distal end, the distal end comprising a bend and an elongate tip (14;16;28;48), wherein the bend places the elongate tip (14;16;28;48) at an angle relative to the straight portion.

11. A combination according to claim 10 wherein the insertion tool (2) comprising two ends (8,12), each end (8,12) comprising an elongate tip (14,16).

12. A combination according to claim 11 wherein each elongate tip (14,16) is angled relative to longitudinal axis of the tool.

13. A combination according to any of claims 10 through 12 wherein an elongate tip (14,16) has a length in the range from 2 to 6 millimeters and a width in the range from 3 to 5 millimeters.

14. A combination according to any of claims 10 through 13 wherein the shaft (6,10) and handle (4) are both straight and are located along a common longitudinal axis.

15. A combination according to claim 10 wherein the insertion tool (2) comprises a medial handle (4), two shafts (6,10) extending from the handle (4) in opposite directions, and elongate cylindrical tips (8,16) at ends (8,12) of each shaft (6,10), the handle (4) and shafts (6,10) being aligned along a common longitudinal axis, the tips (8,16) being angled from the shafts (6,10).

16. A combination according to any of claims 1 through 15 wherein the elongate tip (14,16) is oriented relative to the straight portion (6,10) at an angle in a range from 45 degrees to 170 degrees.

## Patentansprüche

1. Kombination eines Beckenimplantats (50; 70), eines Einführungsinstruments (2; 20; 40; 80) und eines Entfernungsinstruments (82),
wobei das Implantat (50; 70) aufweist
einen Gewebehalteabschnitt,
einen länglichen Erweiterungsabschnitt (52; 72) mit einem proximalen Ende, das am Gewebehalteabschnitt befestigt ist, und einem distalen Ende, und
ein Verbindungsstück (58; 74; 100; 120), das mit dem distalen Ende verknüpft ist,
wobei das Verbindungsstück (58; 74; 100; 120) ein proximales Verbindungsstückende (60; 78; 122) und ein distales Verbindungsstückende (62; 76; 124) aufweist,
wobei das Einführungsinstrument (2; 20; 40; 80) einen länglichen Schaft (6; 10; 24; 44) mit einem distalen Ende aufweist, das konfiguriert ist, mit dem proximalen Ende (60; 78; 122) des Verbindungsstücks (58; 74; 100; 120) in Eingriff zu treten, und
wobei das Entfernungsinstrument (82) ein distales Ende (86; 136) aufweist, das konfiguriert ist, mit dem distalen Ende (62; 76; 124) des Verbindungsstücks (58; 74; 100; 120) in Eingriff zu treten;
**dadurch gekennzeichnet, dass** der längliche Schaft (6; 10; 24; 44) des Einführungsinstruments (2; 20; 40; 80) ein distales Ende aufweist, das einen geraden Abschnitt und eine abgewinkelte längliche Spitze aufweist, wobei die abgewinkelte längliche Spitze konfiguriert ist, mit dem proximalen Ende des Verbindungsstücks in Eingriff zu treten, wenn sich das Verbindungsstück in einer abgewinkelten Ausrichtung bezüglich des geraden Abschnitts befindet.

2. Kombination nach Anspruch 1, wobei
das Verbindungsstück (58; 74; 100; 120) einen länglichen Zylinder (105; 126) aufweist, der eine Längsachse, die sich längs einer Länge des Verbindungsstücks (58; 74; 100; 120) erstreckt, eine proximale Öffnung (132), die sich über einen Abschnitt der Länge vom proximalen Verbindungsstückende (60; 78; 122) erstreckt, und eine distale Öffnung aufweist, die sich über einen Abschnitt der Länge vom distalen Verbindungsstückende (62; 76; 124) erstreckt;
wobei das Einführungsinstrument (2; 20; 40; 80) eine Spitze (130) aufweist, die entfernbar mit der proximalen Öffnung (132) in Eingriff tritt, und das Entfernungsinstrument (82) eine Spitze (86; 136) aufweist, die fest mit der distalen Öffnung (140) in Eingriff tritt.

3. Kombination nach Anspruch 2, wobei die Spitze (86; 136) des Entfernungsinstruments (82) in einer dauerhaften Schnappverbindungsanordnung mit der distalen Öffnung (140) in Eingriff tritt.

4. Kombination nach einem der Ansprüche 1 bis 3, wobei das Verbindungsstück (58; 74; 100; 120) ein sich erweiterndes Verbindungsstück (100) ist, das ein verjüngtes distales Ende (102) aufweist.

5. Kombination nach einem der Ansprüche 2 bis 4, wobei
das Verbindungsstück (58; 74; 100; 120) Seiten (105) aufweist, die sich vom proximalen Verbindungsstückende (104) zum distalen Verbindungsstückende (102) erstrecken, und ferner eine Bohrung (106) aufweist, die sich von einer Seite durch das Verbindungsstück (100) zu einer anderen Seite erstreckt, und
das Implantat (50; 70) eine Hülle (56; 73) aufweist, die mindestens einen Abschnitt des Erweiterungsabschnitts bedeckt, wobei sich die Hülle (56; 73) durch die Bohrung (106) erstreckt und eine Schlinge bildet, um die Hülle (56; 73) am Verbindungsstück (58; 74; 100; 120) zu befestigen.

6. Kombination nach einem der Ansprüche 1 bis 5, wobei das Entfernungsinstrument (82) eine längliche Nadel und ein distales Ende (86; 136) aufweist, das konfiguriert ist, dauerhaft mit einem distalen Ende (62; 76; 124) des Verbindungsstücks (58; 74; 100; 120) in Eingriff zu treten.

7. Kombination nach einem der Ansprüche 1 bis 6, wobei das Einführungsinstrument (2) aufweist
zwei gegenüberliegende Schäfte (6, 10), die jeweils ein Ende (8, 12) aufweisen,
eine erste zylindrische Spitze (14) am ersten Schaftende (8), und
eine zweite zylindrische Spitze (16) am zweiten Schaftende (12).

8. Kombination nach Anspruch 7, wobei die erste Spitze (14) bezüglich des ersten Schaf tendes (8) unter einem Winkel angeordnet ist und die zweite Spitze (16) bezüglich des zweiten Schaftendes (12) abgewinkelt ist, und sich jeder Winkel unterscheidet.

9. Kombination nach Anspruch 8, wobei ein Winkel ein spitzer Winkel ist und ein Winkel ein stumpfer Winkel ist.

10. Kombination nach Anspruch 1, wobei das Einführungsinstrument (2; 20) einen Griff (4; 22; 42), einen Schaft (6; 10; 20; 44), der einen geraden Abschnitt umfasst, und ein distales Ende aufweist, wobei das distale Ende eine Krümmung und eine längliche Spitze (14; 16; 28; 48) aufweist, wobei die Krümmung die längliche Spitze (14; 16; 28; 48) unter einem Winkel bezüglich des geraden Abschnitts anordnet.

11. Kombination nach Anspruch 10, wobei das Einführungsinstrument (2) zwei Enden (8, 12) aufweist, wobei jedes Ende (8, 12) eine längliche Spitze (14, 16) aufweist.

12. Kombination nach Anspruch 11, wobei jede längliche Spitze (14, 16) bezüglich der Längsachse des Instruments abgewinkelt ist.

13. Kombination nach einem der Ansprüche 10 bis 12, wobei eine längliche Spitze (14, 16) eine Länge im Bereich von 2 bis 6 Millimetern und eine Breite im Bereich von 3 bis 5 Millimetern aufweist.

14. Kombination nach einem der Ansprüche 10 bis 13, wobei der Schaft (6, 10) und der Griff (4) beide gerade sind und längs einer gemeinsamen Längsachse angeordnet sind.

15. Kombination nach Anspruch 10, wobei das Einführungsinstrument (2) einen mittleren Griff (4), zwei Schäfte (6, 10), die sich vom Griff (4) in entgegengesetzte Richtungen erstrecken, und längliche zylindrische Spitzen (8, 16) an den Enden (8, 12) jedes Schafts (6, 10) aufweist, wobei der Griff (4) und die Schäfte (6, 10) längs einer gemeinsamen Längsachse ausgerichtet sind, wobei die Spitzen (8, 16) von den Schäften (6, 10) abgewinkelt sind.

16. Kombination nach einem der Ansprüche 1 bis 15, wobei die längliche Spitze (14, 16) bezüglich des geraden Abschnitts (6, 10) unter einem Winkel in einem Bereich von 45 Grad bis 170 Grad ausgerichtet ist.

## Revendications

1. Combinaison d'un implant pelvien (50 ; 70), d'un outil d'insertion (2 ; 20 ; 40 ; 80) et d'un outil de retrait (82), ledit implant (50 ; 70) comprenant
une partie de support de tissu,
une partie d'extension oblongue (52 ; 72) ayant une extrémité proximale fixée à la partie de support de tissu et une extrémité distale, et
un connecteur (58 ; 74 ; 100 ; 120) associé à l'extrémité distale, ledit connecteur (58 ; 74 ; 100 ; 120) comprenant une extrémité proximale (60 ; 78 ; 122) de connecteur et une extrémité distale (62 ; 76 ; 124) de connecteur,
l'outil d'insertion (2 ; 20 ; 40 ; 80) comprenant une tige oblongue (6 ; 10 ; 24 ; 44) ayant une extrémité distale prévue pour s'engager dans l'extrémité proximale (60 ; 78 ; 122) du connecteur (58 ; 74 ; 100 ; 120), et
l'outil de retrait (82) comprenant une extrémité distale (86 ; 136) prévue pour s'engager dans l'extrémité distale (62 ; 76 ; 124) du connecteur (58 ; 74 ; 100 ; 120) ;
**caractérisée en ce que** la tige oblongue (6 ; 10 ; 24 ; 44) de l'outil d'insertion (2 ; 20 ; 40 ; 80) a une extrémité distale comprenant une partie droite et un embout oblong coudé, ledit embout oblong coudé étant prévu pour s'engager dans l'extrémité proximale du connecteur quand le connecteur est orienté suivant un angle par rapport à la partie droite.

2. Combinaison selon la revendication 1, où
le connecteur (58 ; 74 ; 100 ; 120) comporte un cylindre oblong (105 ; 126) comprenant un axe oblong s'étendant sur la longueur du connecteur (58 ; 74 ; 100 ; 120), une ouverture proximale (132) s'étendant sur une partie de la longueur depuis l'extrémité proximale (60 ; 78 ; 122) du connecteur, et une ouverture distale s'étendant sur une partie de la longueur depuis l'extrémité distale (62 ; 76 ; 124) du connecteur ;
l'outil d'insertion (2 ; 20 ; 40 ; 80) comprenant un embout (130) qui s'engage de manière amovible dans l'ouverture proximale (132) et l'outil de retrait (82) comprenant un embout (86 ; 136) qui s'engage de manière inamovible dans l'ouverture distale (140).

3. Combinaison selon la revendication 2, où l'embout (86 ; 136) de l'outil de retrait (82) s'engage dans l'ouverture distale (140) par encliquetage définitif.

4. Combinaison selon l'une des revendications 1 à 3, où le connecteur (58 ; 74 ; 100 ; 120) est un connecteur dilatable (100) comportant une extrémité distale conique (102).

5. Combinaison selon l'une des revendications 2 à 4, où
le connecteur (58 ; 74 ; 100 ; 120) présente des côtés (105) s'étendant de l'extrémité proximale (104) de connecteur à l'extrémité distale (102) de connecteur, et comprend en outre un trou (106) s'étendant dans le connecteur (100) d'un côté à l'autre, et où l'implant (50 ; 70) présente une gaine (56 ; 73) couvrant au moins une partie de la partie d'extension, ladite gaine (56 ; 73) s'étendant dans le trou (106) et formant une boucle pour fixer la gaine (56 ; 73) au connecteur (58 ; 74 ; 100 ; 120).

6. Combinaison selon l'une des revendications 1 à 5, où l'outil de retrait (82) comprend une aiguille oblongue et une extrémité distale (86 ; 136) prévue pour s'engager définitivement dans une extrémité distale (62 ; 76 ; 124) du connecteur (58 ; 74 ; 100 ; 120).

7. Combinaison selon l'une des revendications 1 à 6, où l'outil d'insertion (2) comporte deux tiges (6, 10) opposées, ayant chacune une extrémité (8, 12),
un premier embout cylindrique (14) à la première extrémité (8) de tige, et
un deuxième embout cylindrique (16) à la deuxième extrémité (12) de tige.

8. Combinaison selon la revendication 7, où le premier embout (14) est coudé par rapport à la première extrémité (8) de tige, et où le deuxième embout (16) est coudé par rapport à la deuxième extrémité (12) de tige, chacun suivant un angle différent.

9. Combinaison selon la revendication 8, où un angle est un angle aigu et un angle est un angle obtus.

10. Combinaison selon la revendication 1, où l'outil d'insertion (2 ; 20) comporte une poignée (4 ; 22 ; 42), une tige (6 ; 10 ; 20 ; 44) présentant une partie droite et une extrémité distale, ladite extrémité distale comprenant un coude et un embout oblong (14 ; 16 ; 28 ; 48), le coude pliant l'embout oblong (14 ; 16 ; 28 ; 48) suivant un angle par rapport à la partie droite.

11. Combinaison selon la revendication 10, où l'outil d'insertion (2) comporte deux extrémités (8, 12), chaque extrémité (8, 12) présentant un embout oblong (14 , 16).

12. Combinaison selon la revendication 11, où chaque embout oblong (14 , 16) est coudé par rapport à l'axe longitudinal de l'outil.

13. Combinaison selon l'une des revendications 10 à 12, où un embout oblong (14 , 16) a une longueur comprise entre 2 et 6 millimètres et une largeur comprise entre 3 et 5 millimètres.

14. Combinaison selon l'une des revendications 10 à 13, où la tige (6, 10) et la poignée (4) sont droites toutes les deux et disposées sur un axe longitudinal commun.

15. Combinaison selon la revendication 10, où l'outil d'insertion (2) comprend une poignée centrale (4), deux tiges (6, 10) s'étendant à partir de la poignée (4) dans des directions opposées, et deux embouts cylindriques oblongs (8 , 16), chacun à l'extrémité (8, 12) de chaque tige (6 , 10), la poignée (4) et les tiges (6 , 10) étant alignées sur un axe longitudinal commun, les embouts (8, 16) étant coudés par rapport aux tiges (6 , 10).

16. Combinaison selon l'une des revendications 1 à 15, où l'embout oblong (14 , 16) orienté suivant un angle compris entre 45° et 170° par rapport à la partie droite (6 , 10).
